(19)

Europäisches Patentamt
European Patent Office
Office européen des brevets

(11) **EP 3 335 787 A1**

(12) **EUROPEAN PATENT APPLICATION**

(43) Date of publication:
**20.06.2018 Bulletin 2018/25**

(51) Int Cl.:
**B01J 20/12** [(2006.01)]  **C10G 25/00** [(2006.01)]
**C10G 25/02** [(2006.01)]  **C10G 25/12** [(2006.01)]

(21) Application number: **16203601.6**

(22) Date of filing: **13.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **SCG Chemicals Co., Ltd.**
**Bangkok 10800 (TH)**

(72) Inventor: **Suriye, Kongkiat**
**10270 Samut-Prakan (TH)**

(74) Representative: **Scholz, Volker**
**Boehmert & Boehmert**
**Anwaltspartnerschaft mbB**
**Pettenkoferstrasse 22**
**80336 München (DE)**

(54) **PROCESS FOR REMOVING ALKENE AND/OR ALKYNE FROM A HYDROCARBON FEEDSTOCK**

(57)    The present invention relates to a process for purifying a hydrocarbon feedstock, said process comprising the steps: (a) providing the hydrocarbon feedstock comprising an aromatic compound and at least one compound, selected from the group consisting of alkene, alkyne, nitrogen-containing compound or mixtures thereof; and (b) contacting the hydrocarbon feedstock with an acidic montmorillonite adsorbent at a temperature in the range from 10 to 60 °C.

Figure 1

EP 3 335 787 A1

**Description**

The present invention relates to a process for removing alkene and/or alkyne from a hydrocarbon feedstock comprising an aromatic compound.

It is known that there are various kinds of mixtures of substance containing unsaturated hydrocarbon. Unsaturated hydrocarbon contributes to the bromine index (BI) which is an analytical parameter in order to control the quality of aromatic compound and can be obtained by the amount of bromine in milligrams adsorbed by 100 grams of the sample under given conditions. A low value of BI is preferable to place an aromatic product in the commercial market. Moreover, lower amount of contamination by alkene and/or alkyne or other unsaturated non-aromatic hydrocarbon is normally desired because they could be harmful for a next downstream process utilizing the aromatic compound and can cause undesirable by-products.

It is well known that removing unsaturated non-aromatic hydrocarbon from an aromatic compound may be based on alkylation reaction. Most of prior research and development were focused on an alkylation catalyst starting from activated clay to advanced zeolite.

Such an alkylation reaction causes loss of valuable aromatic compound because it involves reacting the aromatic compound with alkene and/or alkyne to form alkyl aromatic which can normally be easily removed from the aromatic compound by simple distillation.

The clay type catalyst used in the alkylation reaction could not be effectively regenerated because heavy hydrocarbon could be converted to hard coke which is hard to remove at mild temperature resulting in high operation cost. Moreover, a complete removal which has to be performed at high temperature will finally destroy the crystal structure of clay.

US 3,485,884 discloses a method for treating an aromatic hydrocarbon containing mixture by contacting the mixture (contaminated with olefins and sulfur compounds) with aluminum silicate at an elevated temperature within the range of about 200 to about 260 °C followed by distillation. The contacting condition causes alkylation reaction.

US 6,368,496 discloses a method for removing bromine reactive hydrocarbon contaminants from an aromatic stream by providing the aromatic stream having a negligible diene level. The feed stream is then contacted with an active catalyst at conditions including a moderately elevated temperature ranging from about 93 to 260 °C under alkylation reaction to remove unsaturated hydrocarbon.

WO 2011/008344 A1 discloses a method for removing colored-impurities and sulfur-impurities which are bromine index contributors from a hydrocarbon stream by contacting the hydrocarbon stream with an adsorbent that has been pretreated to get a pH about 10.

It is therefore an object of the present invention to provide an improved process for removing alkene and/or alkyne from a hydrocarbon feedstock overcoming drawbacks of the prior art, in particular allowing high efficiency in hydrocarbon purification and consequently reduction of BI value where regeneration is enabled and loss of desirable hydrocarbon is avoided.

The object has been achieved by a process for purifying a hydrocarbon feedstock, said process comprising the steps of providing the hydrocarbon feedstock comprising an aromatic compound, and at least compound selected from the group consisting of alkene, alkyne, nitrogen-containing compound or mixtures thereof; and contacting the hydrocarbon feedstock with an acidic montmorillonite adsorbent at a temperature in the range from 10 to 60 °C, preferably 15 to 35 °C.

While most of terms used herein will be recognizable to those of ordinary skill in the art, it should be understood, however, that when not explicitly defined, terms should be interpreted as adopting a meaning presently accepted by those of ordinary skill in the art. Definition and/or interpretations should not be incorporated from other patent applications, patents, or publications, related or not, unless specifically stated in this specification or if the incorporation is necessary for maintaining validity.

As used herein, the term "hydrocarbon feedstock" refers to an unprocessed stream used to be supplied to a manufacturing process comprising substantially of hydrocarbon, wherein hydrocarbon is any molecule consisting entirely of hydrogen and carbon which could be saturated hydrocarbon, unsaturated hydrocarbon, or aromatic hydrocarbon.

The term "aromatic compound" refers to substances that consist of one or more rings that contain alternating single and double bonds in its chemical structure. In one embodiment, the aromatic compound is an aromatic hydrocarbon compound which preferably consists of one aromatic ring. Preferably, the aromatic compound is an aromatic hydrocarbon compound selected from benzene, toluene, xylene, ethyl benzene, their derivatives, and mixtures thereof, more preferably benzene, toluene, and a mixture thereof.

The term "alkene" refers to any hydrocarbon molecule containing at least one double carbon-carbon bond which can be of aliphatic or cyclic structure. Typically for the present invention, C4 to C10 alkene may be present in the hydrocarbon feedstock.

The term "alkyne" refers to any hydrocarbon molecule containing at least one triple carbon-carbon bond which can be of aliphatic or cyclic structure. Typically for the present invention, C4 to C10 alkyne may be present in the hydrocarbon feedstock.

The term "nitrogen-containing compound" refers to any compound containing at least one nitrogen atom. Preferably for the invention, the nitrogen compound is an organic nitrogen-containing compound, more preferably N-Formyl Morpholine.

As used herein, the term "montmorillonite" refers to a hydrated aluminium silicate clay mineral with expandable structure consisting of two tetrahedral layers of silica sandwiching a central octahedral layer of alumina. Chemically, it is hydrated sodium calcium aluminium magnesium silicate hydroxide. Potassium, iron, and other cations are possible substitutes, and the ratio of cations can be varied. Between layers, there is free-space that is capable to keep the water, cation, or other adsorbates.

Preferably, contacting the hydrocarbon feedstock with the acidic montmorillonite adsorbent is carried out at a gauge pressure in the range from 0 to $6 \times 10^6$ Pascal, preferably 1 to $1 \times 10^6$ Pascal.

In one embodiment, the acidic montmorillonite adsorbent comprises a content of $H^+$ per gram of adsorbent of at least 1 $\mu$mol.

The content of $H^+$ per gram of adsorbent is the number of exchangeable acid sites per gram of the adsorbent. It can be determined by a method involving an aqueous ion exchange of the adsorbent H+ ions with selected cation, followed by titration of the resulting solution.

Particularly, the content of $H^+$ per gram of adsorbent according to the present invention can be determined by the following steps.

(1) Adding 0.2 g of the acidic montmorillonite adsorbent to 10 mL of aqueous solution of 3.4 mol/L NaCl under stirring to perform ion exchange at room temperature;
(2) After 30 hours of ion exchange, solid particle was filtered off;
(3) The filtrate was titrated with 0.05 mol/L of aqueous NaOH solution. The end point of titration (pH is approximately 7) was determined using a pH meter;
(4) The number of exchangeable acid sites in micromol of H+ per gram of adsorbent ($\mu$mol H+/g-adsorbent) was calculated by the following equation (1)

$$\mu mol\ H^+/g\text{-}adsorbent = 10^3\ x\ (volume\ of\ NaOH\ x\ concentration\ of\ NaOH)/g\text{-}adsorbent \quad (1)$$

Details of the steps of determining the content of $H^+$ per gram of adsorbent described above such as amount of adsorbent, type and concentration of ion-exchange solution, type and concentration of titrant could be moidified, or even a different method capable of determining content of $H^+$ per gram of adsorbent known to those with ordinary skill in the art could be used without limitation.

Preferably, for this embodiment, the acidic montmorillonite adsorbent having a content of $H^+$ per gram of adsorbent of at least 1 $\mu$mol is obtained by treating a montmorillonite with an acid.

The acid used for treating the montmorillonite can be any chemical with a pH lower than 7 and is preferably in the form of a solution. Further preferred, the acid is selected from the group consisting of ammonium sulfate, sulfuric acid, hydrochloric acid and mixtures thereof, more preferably ammonium sulfate. Also preferably, the acid has a concentration in the range from 0.5 to 20 percent by weight with respect to the amount of total composition of the solution.

It is preferred that treating the montmorillonite with the acid is performed by contacting the montmorillonite with the acid at a temperature in the range 10 to 60 °C, preferably 15 to 35 °C.

In another embodiment, the acidic montmorillonite adsorbent is characterized by having at least one $NH_3$-TPD desorption peak with the size in the range from 9 to 60 ml/g, preferably 20 to 60 ml/g, at a high desorption region of a temperature in the range of 300 to 800 °C.

$NH_3$-TPD is a method that is extensively used to quantify acid sites of a solid material. Typically, the method involves saturating surface of the solid material with $NH_3$ under certain conditions, followed by linear ramp of temperature in a flowing inert gas stream or connected to vacuum. At higher temperature ammonia is desorbed and the amount of ammonia desorbed is a measure of acid sites on the solid material surface.

Particularly for this invention, the $NH_3$-TPD desorption peak is determined by carrying out $NH_3$-Temperature Programmed Desorption ($NH_3$-TPD) using a TCD detector. Before adsorption, 0.05 g of the acidic montmorillonite adsorbent is dried in a flow of $H_2$ at 425 °C for 2 hours. Adsorption of 10% $NH_3$/He then took place at 50°C until saturation, and then the adsorbent is flushed with He at the same temperature for 2 hours. TCD measurement was carried out from 50 to 800 °C with a heating rate of 10 °C/min using He as a carrier gas. Size of the $NH_3$-TPD desorption peak is calculated from the area of peak.

Preferably for this embodiment, the acidic montmorillonite adsorbent having at least one $NH_3$-TPD desorption peak with the size in the range of 9 to 60 ml/g at a temperature in the range of 300 to 800 °C is an iron-containing montmorillonite. Further preferably, the iron-containing montmorillonite contains $Fe_2O_3$ content in a range from 2 to 20 percent by weight, more preferably 8 to 15 percent by weight with respect to the total weight of the adsorbent.

The iron-containing montmorillonite can be naturally occurred or can be synthesized by ion-exchanging a non-iron montmorillonite with iron containing salt.

In another preferred embodiment, the acidic montmorillonite adsorbent according to the present invention is characterized by having a content of $H^+$/g-adsorbent of at least 1 $\mu$mol and having at least one $NH_3$-TPD desorption peak with the size in the range of 9 to 60 ml/g, preferably 20 to 60 ml/g at a temperature in the range of 300 to 800 °C.

For this embodiment, the acidic montmorillonite adsorbent characterized by having a content of $H^+$/g-adsorbent of at least 1 $\mu$mol and having at least one $NH_3$-TPD desorption peak with the size in the range of 9 to 60 ml/g, preferably 20 to 60 ml/g at a temperature in the range of 300 to 800 °C is preferably obtained by treating an iron-containing montmorillonite with an acid. More preferably, the acidic montmorillonite adsorbent characterized by having a content of $H^+$/g-adsorbent of at least 1 $\mu$mol and having at least one $NH_3$-TPD desorption peak with the size in the range of 9 to 60 ml/g, preferably 20 to 60 ml/g at a temperature in the range of 300 to 800 °C is obtained by treating the iron-containing montmorillonite containing $Fe_2O_3$ content in a range from 2 to 20 percent by weight, even more preferably 8 to 15 percent by weight with respect to the total weight of the montmorillonite with an acid selected from the group consisting of ammonium sulfate, sulfuric acid, hydrochloric acid and mixtures thereof, more preferably ammonium sulfate.

Treating the iron-containing montmorillonite with the acid is preferably carried out in the manner already described above.

Alternatively, the acidic montmorillonite adsorbent characterized by having a content of $H^+$/g-adsorbent of at least 1 $\mu$mol and having at least one $NH_3$-TPD desorption peak with the size in the range of 9 to 60 ml/g, preferably 20 to 60 ml/g, at a temperature in the range of 300 to 800 °C may also obtained by treating a montmorillonite with an acid first and followed by ion-exchanging the acid treated montmorillonite with Fe-containing salt.

Surprisingly, it was found that the acidic montmorillonite adsorbent according to the invention can selectively adsorb alkene and/or alkyne from a hydrocarbon feedstock comprising an aromatic compound at mild temperature, i.e. below 60°C.

It was also observed that a nitrogen-containing compound contaminant was significantly removed from the hydrocarbon feedstock comprising an aromatic compound by the inventive process.

It was further surprisingly found that, the inventive process does not cause the formation of any by-product and no loss of aromatic compound in the hydrocarbon feedstock was observed.

Preferably, contacting the hydrocarbon feedstock with the acidic montmorillonite adsorbent is carried out at a weight hourly space velocity in the range from 0.01 to 30 hour$^{-1}$, more preferably from 0.1 to 15 hour$^{-1}$, and even more preferably from 1 to 7 hour$^{-1}$.

The inventive process can further comprise, after contacting the hydrocarbon feedstock with the acidic montmorillonite adsorbent, a step of regenerating the acidic montmorillonite adsorbent which is deactivated or saturated by the adsorbed alkene and/or alkyne. The step of regenerating is carried out by contacting the deactivated acidic montmorillonite adsorbent with an inert gas at a temperature in the range from 100 to 400 °C, preferably 150 to 300 °C, and more preferably 190 to 200 °C.

Preferably, the inert gas used in the step of regenerating is selected from nitrogen, helium, and argon, preferably nitrogen.

Due to mild condition employed by the inventive process, it was found that the adsorbent can easily be regenerated to remove polymer and/or carbon deposited on the surface thereof without destroying its structure. This provides the further advantage of producing much lower solid waste of used adsorbent generated per year.

The invention is now further illustrated on the basis of Examples from which further features and advantages may be taken. It is to be noted that the following explanations are presented for the purpose of illustrating only; they are not intended to be exhaustive or to limit the invention to the precise form disclosed.

Example

The following adsorbents were applied in the below examples;

Adsorbent "A" is a commercially available acid-treated montmorillonite adsorbent according to the invention.

Adsorbent "B" is an unmodified natural montmorillonite adsorbent (comparative example).

Adsorbent "C" is an unmodified natural acidic montmorillonite adsorbent according to the invention.

Adsorbent "D" is an acid treated montmorillonite adsorbent obtained by impregnation of 2 wt% of $(NH_4)_2SO_4$ over the Adsorbent "C" following by drying at 120°C for 3 hours, according to the invention.

Characterization properties of each adsorbent are shown in the following Table 1.

Table 1

| Adsorbent | Characterization Property | | | | |
|---|---|---|---|---|---|
| | NH3-TPD Desorption peak size at low temp. (below 300°C) [ml/g at STP] | NH3-TPD Desorption peak size at high temp. (below 300-800 °C) [ml/g at STP] | H+/g-adsorbent [$\mu$mol] | pH | $Fe_2O_3$ content [%wt] |
| A | > 43.1 | <8.7 | >500 | 3.3 | 1.56 |
| B | >15.7 | <16.6 | <1 | 0 | 1.6 |
| C | >14.6 | >41.4 | <1 | 0 | 12 |
| D | >30.2 | >33.5 | >50 | 4.3 | 7.3 |

**Example 1:** Removing cyclopentene from benzene

Each sample adsorbent was placed into the oven in order to remove trace of water at temperature 180°C for overnight before testing.

The hydrocarbon feedstock used for testing was 150 ppm of cyclopentene in 0.05 grams of benzene.

Each adsorbent (previously dried) was added into the 25-mL of the hydrocarbon feedstock and was continuously stirred for 9 hours.

The resulting cyclopentene/benzene sample was then analyzed by Gas Chromatography - Flame Ionization Detector (GC-FID), which is a very common analytical technique that is widely used in the petrochemical to see the amount of cyclopentene left in the benzene sample.

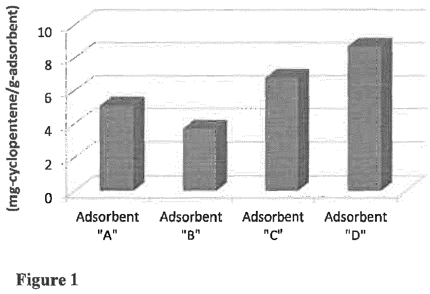

Results of adsorption test for Adsorbent "A", "B", "C", and "D" are displayed in Figure 1.

**Example 2** The Adsorbent "A", "B", "C", and "D" were tested for adsorption of nitrogen compound. The test was perform by packing 20 g of each adsorbent , previously dried at 180 °C in an oven overnight, into separate cylinder vessels. The industrial benzene feedstock, containing N-Formyl Morpholine (NFM) as a representative of a nitrogen compound, was then flowed pass each of the packed adsorbent at room temperature and ambient pressure. The inlet and outlet benzene concentration were analyzed by GC-FID.

Result of this test is shown in Table 2.

Table 2

| | Inlet NFM [ppb] | Outlet NFM [ppb] |
|---|---|---|
| Adsorbent A | 1020 | 104 |
| Adsorbent B | | 332 |
| Adsorbent C | | 16 |
| Adsorbent D | | Non detectable |

From the result above, it can be seen that the inventive adsorbents can also be used for adsorbing a nitrogen compound, which is oftentimes found contaminated in benzene stream.

**Example 3:** Reduction of BI value in benzene

The Adsorbent "D" was used to test its efficiency in BI reduction in industrial benzene feedstock. 20 g of Adsorbent "D", previously dried at 180 °C in an oven overnight, was pack into a cylinder vessel. The industrial benzene feedstock was then flowed pass the packed adsorbent at room temperature and ambient pressure. Inlet and outlet BI value of the industrial benzene were measured according to the standard method ASTM 5776-99. The inlet and outlet benzene concentration were analyzed by GC-FID. The test was repeated 3 times.

Results of this test are displayed in the following Table 2. It can be seen from the results that the Adsorbent "D" can efficiently reduce BI value in benzene feedstock to a very low level. Moreover, no loss of benzene was observed.

Table 3

| 1st | 1st Test | | 2nd Test | | 3rd Test | |
|---|---|---|---|---|---|---|
| | Inlet | Outlet | Inlet | Outlet | Inlet | Outlet |
| Bromine Index (BI) Value | 10.10 | 0.4 | 11.55 | 0.9 | 12.87 | 0.5 |
| Benzene (wt%) | 99.9316 | 99.9532 | 99.9275 | 99.9536 | 99.9063 | 99.9268 |

The features described in the foregoing description and in the claims may, both separately and in any combination, be material for realizing the invention in diverse forms thereof.

**Claims**

1. A process for purifying a hydrocarbon feedstock, said process comprising the steps:

   (a) providing the hydrocarbon feedstock comprising an aromatic compound and at least one compound, selected from the group consisting of alkene, alkyne, nitrogen-containing compound or mixtures thereof; and
   (b) contacting the hydrocarbon feedstock with an acidic montmorillonite adsorbent at a temperature in the range from 10 to 60 °C.

2. The process according to claim 1, wherein the aromatic compound is an aromatic hydrocarbon compound selected from benzene, toluene, xylene, ethylbenzene, their derivatives, and mixtures thereof.

3. The process according to claim 1 or 2, wherein the alkene is C4 to C10 alkene.

4. The process according to any preceding claim, wherein the alkyne is C4 to C10 alkyne.

5. The process according to any preceding claim, wherein the nitrogen-containing compound is N-formyl morpholine.

6. The process according to any preceding claim, wherein contacting of the hydrocarbon feedstock with the acidic montmorillonite adsorbent is carried out at a gauge pressure in the range from 0 to $6\times10^6$ Pascal.

7. The process according to any preceding claim, wherein the acidic montmorillonite adsorbent comprises a content of $H^+$ per gram of adsorbent of at least 1 $\mu$mol.

8. The process according to claim 7, wherein the acidic montmorillonite adsorbent is obtainable by treating a montmorillonite with an acid.

9. The process according to claim 8, wherein the acid is selected from the group consisting of ammonium sulfate, sulfuric acid, hydrochloric acid, and mixtures thereof.

10. The process according to any of the preceding claims, wherein the acidic montmorillonite adsorbent is **characterized by** having at least one $NH_3$-TPD desorption peak with the size in the range from 9 to 60 ml/g at a high desorption region of a temperature in the range of 300 to 800 °C.

11. The process according to claim 10, wherein the acidic montmorillonite adsorbent is an iron-containing montmorillonite.

12. The process according to claim 11, wherein the iron-containing montmorillonite has a $Fe_2O_3$ content in a range from 2 to 20 percent by weight with respect to the total weight of adsorbent.

13. The process according to any of the preceding claims, wherein the process further comprises, after contacting the hydrocarbon feedstock with the acidic montmorillonite adsorbent, a step of regenerating the acidic montmorillonite adsorbent.

14. The process according to claim 13, wherein the step of regenerating comprises contacting the acidic montmorillonite adsorbent with an inert gas at a temperature in the range from 100 to 400 °C.

15. The process according to claim 14, wherein the inert gas is selected from nitrogen, helium, and argon.

Figure 1

**EP 3 335 787 A1**

Europäisches Patentamt

European Patent Office

Office européen des brevets

## EUROPEAN SEARCH REPORT

Application Number

EP 16 20 3601

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2013/267742 A1 (MINOUX DELPHINE [BE] ET AL) 10 October 2013 (2013-10-10)<br>* paragraphs [0048] - [0050], [0143], [0189], [0190], [0197] *<br>* claims 1,3,4,8 *<br>* tables 4-8 *<br>* examples 3,5 *<br><br>----- | 1-4,6-12<br><br>13-15<br>5 | INV.<br>B01J20/12<br>C10G25/00<br>C10G25/02<br>C10G25/12 |
| X<br><br><br><br><br><br><br><br><br><br><br><br>Y<br>A | Kirsi Linnavuori: "TECHNO-ECONOMIC ASSESSMENT OF THE REMOVAL OF BENZENE IMPURITIES",<br>Master's thesis for the degree of Master of Science in Technology,<br>22 September 2016 (2016-09-22),<br>XP055382042,<br>Espoo, Finland<br>Retrieved from the Internet:<br>URL:https://aaltodoc.aalto.fi/bitstream/handle/123456789/22135/master_Linnavuori_Kirsi_2016.pdf?sequence=1<br>[retrieved on 2017-06-15]<br>* chapter 3.1.1 *<br>* pages 42-48: chapters 10, 10.1-10.3 *<br>* Appendix 1 and 2 *<br>& Kirsi Linnavuori: "Techno-economic assessment of the removal of benzene impurities - Library Record",<br>Master's thesis for the degree of Master of Science in Technology,<br>22 September 2016 (2016-09-22),<br>XP055382045,<br>Espoo, Finland<br>Retrieved from the Internet:<br>URL:https://aaltodoc.aalto.fi/handle/123456789/22135?show=full<br>[retrieved on 2017-06-15]<br><br>-----<br><br>-/-- | 1,2,5-8,<br>10-12<br><br><br><br><br><br><br><br><br><br>13-15<br>3,4,9 | TECHNICAL FIELDS SEARCHED (IPC)<br><br>C10G<br>B01J<br>C07C |

The present search report has been drawn up for all claims

| Place of search | Date of completion of the search | Examiner |
|---|---|---|
| The Hague | 15 June 2017 | Baumlin, Sébastien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 16 20 3601

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/051023 A1 (RELIANCE IND LTD [IN]; PURANIK VIJAYALAKSHMI RAVI [IN]; KUMAR PRAKASH) 11 April 2013 (2013-04-11) | 1-3,6-12 | |
| Y | * claims 1,3,6,14,15,18 * | 13-15 | |
| A | * page 10, lines 1-15 *<br>* examples 1-3 *<br>----- | 4,5 | |
| Y | US 4 425 226 A (REUSSER ROBERT E [US] ET AL) 10 January 1984 (1984-01-10) | 13,14 | |
| A | * column 4, lines 48-68 *<br>* claims 1,8 *<br>* examples III, I *<br>----- | 1-12 | |
| A,D | WO 2011/008344 A1 (GTC TECHNOLOGY US LLC [US]; SEALEY AMY [US]; WYTCHERLEY RANDI [US]) 20 January 2011 (2011-01-20)<br>* claims 1-6 *<br>* example 2 *<br>----- | 1-15 | |
| A | US 2004/186017 A1 (FLESSNER UWE [DE]) 23 September 2004 (2004-09-23)<br>* example 4 *<br>* claims 1,7,9 *<br>----- | 1-15 | TECHNICAL FIELDS SEARCHED (IPC) |

The present search report has been drawn up for all claims

| Place of search | Date of completion of the search | Examiner |
|---|---|---|
| The Hague | 15 June 2017 | Baumlin, Sébastien |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.....................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 20 3601

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2017

| Patent document cited in search report | | Publication date | Patent family member(s) | | Publication date |
|---|---|---|---|---|---|
| US 2013267742 | A1 | 10-10-2013 | BR 112013014763 A2 | | 04-10-2016 |
| | | | CN 103282465 A | | 04-09-2013 |
| | | | EP 2658952 A1 | | 06-11-2013 |
| | | | KR 20140019318 A | | 14-02-2014 |
| | | | US 2013267742 A1 | | 10-10-2013 |
| | | | WO 2012089716 A1 | | 05-07-2012 |
| WO 2013051023 | A1 | 11-04-2013 | NONE | | |
| US 4425226 | A | 10-01-1984 | NONE | | |
| WO 2011008344 | A1 | 20-01-2011 | CN 101955791 A | | 26-01-2011 |
| | | | EP 2454345 A1 | | 23-05-2012 |
| | | | KR 20120051701 A | | 22-05-2012 |
| | | | RU 2012105430 A | | 27-08-2013 |
| | | | TW 201103888 A | | 01-02-2011 |
| | | | US 2011011770 A1 | | 20-01-2011 |
| | | | WO 2011008344 A1 | | 20-01-2011 |
| US 2004186017 | A1 | 23-09-2004 | AT 302059 T | | 15-09-2005 |
| | | | DE 10127927 A1 | | 12-12-2002 |
| | | | EP 1397203 A1 | | 17-03-2004 |
| | | | ES 2247334 T3 | | 01-03-2006 |
| | | | JP 2004532122 A | | 21-10-2004 |
| | | | JP 2008132488 A | | 12-06-2008 |
| | | | PT 1397203 E | | 30-11-2005 |
| | | | US 2004186017 A1 | | 23-09-2004 |
| | | | WO 02100533 A1 | | 19-12-2002 |

EPO FORM P0459

For more details about this annex : see Official Journal of the European Patent Office, No. 12/82

**EP 3 335 787 A1**

**Patent documents cited in the description**

- US 3485884 A
- US 6368496 B

- WO 2011008344 A1